# EUROPEAN PATENT APPLICATION

(11) **EP 4 752 549 A1**
(43) Date of publication of application: **03.06.2026**
(21) Application number: 24216639.5
(22) Date of filing: 29.11.2024
(51) Int. Cl.: G01N 33/68, G01N 33/94

(54) **BIOMARKER FOR TREATMENT RESPONSE IN MAJOR DEPRESSIVE DISORDER**

(71) Applicant: Karl-Franzens-Universität Graz, 8010 Graz (AT)
(72) Inventor: The designation of the inventor has not yet been filed
(74) Representative: SONN Patentanwälte GmbH & Co KG

(57) **Abstract**

Disclosed is the use of spermidine as a biomarker for monitoring the treatment response in major depressive disorder (MDD). Also disclosed are methods for assessing a response to an MDD treatment in a patient having MDD, antidepressants for use in treatment of MDD and methods of treating MDD.

## Description

### FIELD OF THE INVENTION

The field of the present invention relates to biomarkers for assessing treatment responses in major depressive disorder (MDD) .

### BACKGROUND

MDD is a prevalent and debilitating mental health condition generally characterized by persistent feelings of sadness, hopelessness, and a loss of interest or pleasure in nearly all activities (anhedonia). MDD affects millions of individuals worldwide and is a leading cause of disability and suicide, contributing significantly to the global burden of disease. The aetiology of MDD is multifactorial, involving genetic, neurobiological, environmental, and psychological factors. Despite extensive research, the precise pathophysiological mechanisms underlying MDD remain incompletely understood. On a molecular level, multiple signalling pathways are believed to be involved (Fries, et al, 2022).

Current treatment modalities for MDD include pharmacotherapy, psychotherapy, and neuromodulation therapies.

Pharmacotherapy primarily involves the administration of antidepressant medications such as selective serotonin reuptake inhibitors (SSRIs), serotonin-norepinephrine reuptake inhibitors (SNRIs), atypical antidepressants, tricyclic antidepressants (TCAs), and monoamine oxidase inhibitors (MAOIs). These antidepressants may be augmented by antipsychotics, anticonvulsants or lithium. In addition, N-methyl-D-aspartate (NMDA) receptor antagonists such as ketamine are emerging as new pharmacotherapies.

Psychotherapy encompasses various evidence-based approaches, including cognitive-behavioural therapy (CBT), interpersonal therapy (IPT), and psychodynamic therapy.

Neuromodulation therapies involve techniques such as electroconvulsive therapy (ECT), transcranial magnetic stimulation (TMS), transcranial direct current stimulation (tDCS), vagus nerve stimulation (VNS), and deep brain stimulation (DBS), which modulate neural activity through electrical or magnetic means.

Despite the availability of these treatments, achieving remission in MDD remains challenging. Approximately two thirds of patients fail to achieve remission upon first-line treatment according to current clinical guidelines. The likelihood of remission decreases with subsequent treatment attempts, leading to a condition known as treatment-resistant depression (TRD), see e.g. Fava 2003. TRD is associated with increased morbidity, higher healthcare costs, and a significant impact on quality of life.

Another complication is that MDD severity is typically assessed with clinician-rated or self-rated questionnaires. For instance, the Hamilton Depression Rating Scale (HAMD-17; see Hamilton, 1960) is a widely recognized clinical tool designed to assess the severity of depressive symptoms in individuals diagnosed with MDD. This scale comprises 17 items (hence "HAMD-17") that evaluate various aspects of depression, including mood, feelings of guilt, suicidal ideation, insomnia, work and activities, psychomotor retardation, agitation, anxiety (both psychological and somatic), somatic symptoms (such as gastrointestinal issues), hypochondriasis, weight loss, and insight. Each item is scored on a scale ranging from 0 to 2 or 0 to 4, with higher scores indicating more severe depressive symptoms.

The interpretation of HAMD-17 scores typically follows this guideline: scores between 0 and 7 suggest no depression; scores from 8 to 13 indicate mild depression; scores ranging from 14 to 19 signify moderate depression; scores between 20 and 25 reflect severe depression; and scores of 26 or higher denote very severe depression.

The Inventory of Depressive Symptomatology (IDS) is another instrument used to assess the severity of depressive symptoms in individuals with MDD (Rush et al, 1996). The IDS is available in two formats: the clinician-rated version (IDS-C) and the self-report version (IDS-SR). Both versions as used herein (and disclosed in detail in Rush et al, 2000) have 30 items that evaluate a wide range of depressive symptoms, including mood disturbances, cognitive impairments, anxiety, irritability, sleep patterns, appetite changes, and psychomotor activity.

Each item on the IDS is scored on a scale from 0 to 3, with higher scores indicating more severe symptoms. The total score can range from 0 to 84, providing a comprehensive assessment of the patient's depressive state. Interpretation of the IDS scores typically follows these guidelines: scores from 0 to 13 suggest no depression; scores between 14 and 25 indicate mild depression; scores from 26 to 38 signify moderate depression; scores between 39 and 48 reflect severe depression; and scores of 49 or higher denote very severe depression.

The IDS-C is administered by trained healthcare professionals who evaluate the patient's symptoms based on a structured interview, aiming to capture the clinician's observations and assessments. The IDS-SR allows patients to self-report their symptoms, providing personal insights into their condition.

The IDS-SR relies heavily on the patient's self-awareness and honesty, which can be affected by the nature of the disorder.

The HAMD-17 and IDS-C are generally administered by trained healthcare professionals, such as psychiatrists or psychologists. Despite being standardized, questionnaire-based scoring can still be influenced by the clinician's personal interpretation, making it somewhat subjective. Additionally, scoring can be time-consuming and requires a trained professional to at least over some level of objectivity.

Biomarkers have shown potential for a more objective and less laborious assessment of MDD severity and the course of treatment than traditional assessments. Currently, many potential biomarkers are being researched for their usefulness in identifying, diagnosing, and monitoring depression. These include genetic markers, such as specific gene variants related to neurotransmitter function and stress response pathways. Inflammatory markers, such as cytokines and C-reactive protein, have been associated with depressive states, suggesting a link between inflammation and MDD pathophysiology. In addition, neuroimaging techniques, including functional magnetic resonance imaging (fMRI) and positron emission tomography (PET), are utilized to explore structural and functional changes in the brain associated with depression.

For instance, Strawbridge et al, 2017, gives an overview about biomarkers for depression.

You et al., 2024, disclose a metabolomic analysis on the contribution of polyamine pathway to determinations of diagnosis for TRD. Spermine, spermidine, and carnosine were considered the most promising biomarkers for diagnosing TRD and first-episode drug-naive MDD patients, based on machine-learning conducted on small patient groups.

While diagnostic biomarkers are still under investigation and not yet widely implemented in clinical practice, they hold promise for enhancing the diagnosis of depression.

By contrast, biomarkers that allow for effective differentiation between responders and non-responders to MDD treatments are not well researched, let alone clinically established. This presents a significant challenge, as the heterogeneity of MDD means that patients may respond differently to the same treatment. The inability to monitor treatment outcomes with the required level of objectivity leads to an undesirable trial-and-error approach, prolonging patient suffering and delaying effective intervention.

Targum et al., 2022, teaches that increased prostaglandin E1 stimulated adenylyl cyclase was associated with antidepressant response in MDD subjects, based on a small group of MDD patients observed (11 antidepressant responders, 8 non-responders) .

US 2022/0187315 A1 relates to methods and compositions for diagnosing and evaluating the treatment of depression using one or more biomarkers for the diagnosis and monitoring treatment efficacy. More than hundred metabolites are suggested as potential biomarkers in this document, mostly based on inconclusive experimental results.

There is still an unmet need for biomarkers that enable reliable monitoring of the treatment response, allowing for personalized treatment strategies that improve clinical outcomes and reduce the time to remission.

### SUMMARY OF THE INVENTION

An object of the present invention is thus to provide biomarkers for monitoring the treatment response in MDD.

The present invention provides the use of spermidine as a biomarker (from blood, in particular plasma, saliva or urine) for monitoring the treatment response in MDD. Preferably, the spermidine/putrescine ratio is used.

In another aspect, the present invention provides a method for assessing (or: monitoring) a response to an MDD treatment in a patient having MDD. The method comprises the following steps:
a) determining a first spermidine level in a first sample of the patient, wherein the first sample has been obtained from the patient prior to the MDD treatment,
b) determining a second spermidine level in a second sample of the patient, wherein the second sample has been obtained while the patient is undergoing the MDD treatment or has undergone the MDD treatment, and
c) correlating the first spermidine level with the second spermidine level to assess (or: monitor) the response to the MDD treatment.

The method preferably comprises d) designating the patient as a responder to the MDD treatment if said correlating found a spermidine level increase, and/or designating the patient as a non-responder to the MDD treatment if said correlating found a spermidine level decrease or unchanged spermidine level.

In yet another aspect, antidepressant for use in treatment of MDD, wherein the spermidine level is used as a biomarker for treatment response, is provided. The treatment response is preferably assessed (or: monitored) by the method disclosed above.

In a final aspect, the present invention provides a method for treating MDD. This method comprises the following steps:
a) obtaining a first sample from a patient having MDD,
b) determining a first spermidine level in the first sample,
c) administering an MDD treatment (e.g., an effective amount of an antidepressant) to the patient,
d) obtaining a second sample from the patient,
e) determining a second spermidine level in the second sample, and
f) correlating the first spermidine level with the second spermidine level to assess (or: monitor) a response to the MDD treatment;

In this method, step a) is performed before step c) and step d) is performed after or concurrently with step c) (i.e., if step c) has a longer duration).

Typically, if said correlating indicates no clinical improvement of MDD, the patient is switched to another MDD treatment (e.g., from an SSRI to an SNRI) or an augmentation strategy is applied, and/or, if said correlating indicates clinical improvement of MDD, the MDD treatment is continued as is.

In embodiments, the method may comprise the step of designating the patient as a responder to the MDD treatment if said correlating found a spermidine level increase, and/or designating the patient as a non-responder to the MDD treatment if said correlating found a spermidine level decrease or unchanged spermidine level.

In the course of the present invention, spermidine turned out to be a reliable biomarker for monitoring MDD treatment response, in the context of a clinical study with MDD patients undergoing antidepressant treatment. By contrast, another polyamine, namely putrescine, turned out to be unsuitable for differentiating between responders and non-responders on its own during MDD treatment. Finally, the spermidine/putrescine ratio also turned out as suitable for assessing the response to MDD treatment.

Accordingly, in all of the above aspects, it is preferred that additionally, a first and second putrescine level (in the first and second samples, respectively) is determined to obtain a first and a second spermidine/putrescine ratio. In other words, in the context of the present invention, the "spermidine level" may preferably be defined as the spermidine level normalized to the respective putrescine level, i.e. defined as the "spermidine/putrescine ratio". Evidently, both molar and mass ratios may be used.

The methods of the present invention may comprise further method steps. The skilled person is aware that the order of the method steps may be changed if it is technically feasible (and not explicitly stated otherwise).

The following detailed description and preferred embodiments apply to all aspects of the invention and can be combined with each other without restriction, except were explicitly indicated.

### DETAILED DESCRIPTION OF THE INVENTION

In recent years, there has been growing interest in the role of polyamines in neuropsychiatric disorders, particularly MDD. Emerging evidence suggests that dysregulation of polyamine metabolism may be associated with the development of MDD.

Unrelated to the monitoring of MDD treatment response, Qi et al., 2024, concerns an observational study on the association between dietary spermidine intake and depressive symptoms among US adults. A negative association between dietary spermidine intake and depression was found.

The primary polyamines - putrescine, spermidine, and spermine - are derived from amino acids. Putrescine is the simplest of these polyamines and serves as a precursor for the synthesis of spermidine and spermine. It is formed by the decarboxylation of ornithine, a process catalysed by the enzyme ornithine decarboxylase. Spermidine is synthesized from putrescine, a reaction mediated by the enzyme spermidine synthase. In turn, spermidine is the precursor of spermine and a substrate for the enzyme spermine synthase. The levels of these polyamines may be quantified in the blood (in particular plasma), urine and saliva of patients by routine methods. A preferred method for determination of polyamines in biological samples is disclosed in Magnes et al, 2014.

In a preferred embodiment, both the first sample and the second sample are selected from the group consisting of a blood sample, a urine sample, and a saliva sample. More preferably, both the first sample and the second sample are plasma samples. While plasma samples are particularly suitable for achieving higher reliability, urine and saliva samples have the advantage that they may be collected by the patients themselves.

In a preferred embodiment, the time interval between the collection of the first sample and the second sample is at least one week. More preferably, this interval is at least two weeks, even more preferably at least three weeks, and especially at least four weeks (or even at least six weeks, or even at least eight weeks). This allows for a more meaningful assessment of changes in spermidine levels over time. By temporally spacing the samples adequately, this approach ensures that transient fluctuations are minimized, and genuine trends indicative of treatment response may be more accurately detected, thereby enhancing the reliability of the monitoring method. Of course, during treatment of the patient's MDD, further samples may be taken to monitor the spermidine level.

In another preferred embodiment, the time interval between the initiation of MDD treatment (e.g., the first administration of an antidepressant or of a neuromodulation therapy) and the collection of the second sample is at least one week. More preferably, this interval is at least two weeks, even more preferably at least three weeks, and especially at least four weeks (or even at least six weeks, or even at least eight weeks). This is advantageous as it increases the likelihood that the patient has been undergoing treatment for a sufficient period to allow for more reliable detection of changes in spermidine levels, which reflect the response to the therapy. By relying on these longer time intervals, the effectiveness of monitoring and decision basis any necessary adjustments to the treatment plan is further improved.

In a preferred embodiment, an increase in spermidine level (or: spermidine/putrescine ratio) is indicative of a positive treatment response, specifically a clinical improvement in MDD. This clinical improvement is advantageously defined as a decrease in the Hamilton Depression Rating Scale (HAMD)-17 score (from baseline), or in the IDS-C or IDS-SR score. Particularly, a decrease of at least 20%, preferably at least 30%, more preferably at least 40%, especially at least 50% (from baseline) indicates such improvement.

In another preferred embodiment, a decrease in spermidine level (or the spermidine level remaining unchanged) indicates that the treatment response does not correspond to a clinical improvement in MDD. The same may apply to the spermidine/putrescine ratio. Specifically, this lack of clinical improvement is defined by the HAMD-17 score decreasing by less than 20% (from baseline), remaining unchanged (from baseline), or even increasing (from baseline). Alternatively, this lack of clinical improvement may be defined by the IDS-C or IDS-SR score decreasing by less than 20% (from baseline), remaining unchanged (from baseline), or even increasing (from baseline).

In another preferred embodiment, the MDD treatment (as monitored by the method or use of the present invention) is selected from the group consisting of pharmacological therapy, neuromodulation therapies, and psychotherapy.

In a preference, the pharmacological therapy is with an antidepressant selected from the group consisting of SSRIs, SNRIs, serotonin modulators, MAOIs, tricyclic antidepressants, atypical antidepressants and NMDA receptor antagonists (or the antidepressant for use according to the invention is preferably selected from this group).

SSRIs enhance serotonergic neurotransmission by increasing serotonin levels; preferred SSRIs are fluoxetine, sertraline, paroxetine, citalopram, escitalopram, fluvoxamine, vilazodone, and vortioxetine.

SNRIs elevate both serotonin and norepinephrine levels, with venlafaxine, desvenlafaxine, duloxetine, levomilnacipran, and milnacipran being preferred.

Serotonin modulators act on various serotonin receptors to modulate neurotransmission and may also inhibit serotonin reuptake. Preferred antidepressants include trazodone, nefazodone, vilazodone, and vortioxetine.

MAOIs inhibit the breakdown of monoamine neurotransmitters, thereby increasing their levels; phenelzine, tranylcypromine, isocarboxazid, selegiline, and moclobemide are preferred.

Tricyclic antidepressants inhibit the reuptake of norepinephrine and serotonin and affect other neurotransmitter systems as well. Preferred tricyclic antidepressants are amitriptyline, nortriptyline, imipramine, desipramine, clomipramine, doxepin, protriptyline, trimipramine, amoxapine, and maprotiline.

Atypical antidepressants often target multiple neurotransmitter systems; preferred are bupropion, mirtazapine and agomelatine.

NMDA receptor antagonists represent a rather recent approach by modulating glutamatergic neurotransmission, which is generally associated with rapid antidepressant effects. Preferred agents in this category include esketamine, ketamine, and dextromethorphan (may be used e.g., in combination with bupropion).

In the context of the present invention, the patient has MDD (as e.g., defined by the Diagnostic and Statistical Manual of Mental Disorders, 5th edition - DSM-5), preferably non-psychotic MDD. Preferably, the patient is an adult, in particular from 18 - 65 years old. The present invention is particularly useful if the patient is an outpatient, as these patients may not be monitored and scored by traditional methods as easily as hospitalised patients. It is further preferred that the patient is non-suicidal.

In another preferred embodiment, the MDD is further defined by a HAMD-17 score of at least 18. In other words, the present invention is particularly useful for moderately severe, severe and very severe MDD. Alternatively, or in addition thereto, the MDD is further defined by an IDS-C score of at least 20.

Herein, the uses and methods for assessing (or: monitoring) a response to an MDD treatment disclosed are preferably in vitro methods, i.e. they preferably do not comprise surgical, diagnostic or therapeutic method steps.

### EXAMPLES

The present invention is further described by the following figure and example, to which it is not restricted.

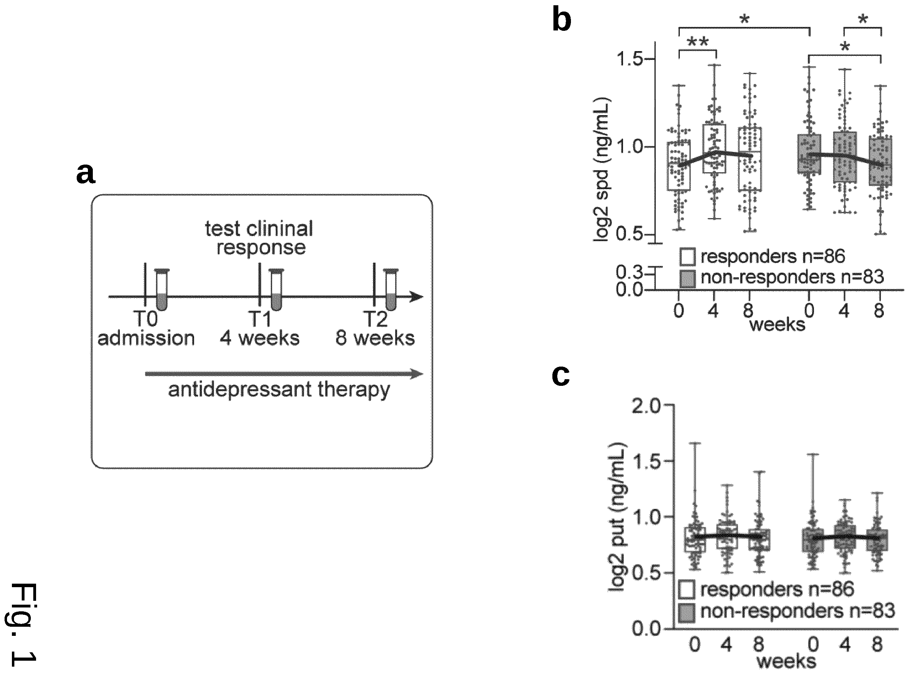

**Fig. 1** **- Change of plasma spermidine, but not plasma putrescine alone, indicates treatment response.** (a) Schematic overview of the study design for the cohort involving depressed patients, including the time points for blood sample collection and the duration of antidepressant treatment. (b) Targeted analysis of the biogenic polyamine spermidine in depressed patients from this cohort, with measurements taken at baseline (T0/BL), after 4 weeks (W4), and after 8 weeks (W8) of antidepressant treatment. The cohort is divided into responders (n = 86) and non-responders (n = 83) according to changes in HAM-D 17 from BL to W4, with the results highlighting the comparison of spermidine levels between these groups. In responders, the spermidine level is significantly increased already at the W4 timepoint. By contrast, in non-responders, the spermidine level was essentially unchanged at W4 and even significantly decreased at W8. p ≤ 0.05 (*), p ≤ 0.01 (**). (c) When the same analysis was applied to the biogenic polyamine putrescine, no significant changes at W4 and W8 were observed in the two groups of the same cohort.

### Example

### Study design

All patients were enrolled in the "Randomized clinical trial comparing an early medication change (EMC) with treatment as usual (TAU) in patients with depression - the EMC trial" (ClinicalTrials.gov identifier: NCT00974155). A total of 889 depressed patients were enrolled in the study between 2009 and 2014. The detailed treatment algorithm was published in Tadic et al, 2010. In summary, the EMC trial was a multicenter, randomized, controlled clinical trial, which investigated whether patients with no improvement after 14 days of escitalopram treatment would benefit from an early medication switch (EMC: switch to venlafaxine from day 14, followed by augmentation with lithium if again nonresponsive on day 28) compared with patients treated according to current guidelines (TAU: continue escitalopram for two weeks and switch to venlafaxine on day 28). The main inclusion criteria for the EMC trial were: (1) depression, first episode or recurrent, according to DSM-IV, (2) a HAM-D 17 score of 18 at screening, (3) age 18-65 years and 60 years at the time of the first depressive episode. Main exclusion criteria: (1) bipolar disorder or psychotic depression, (2) benzodiazepines >1.5 mg lorazepam per day, (3) non-native speaker of German, (4) adequate (dose and time) treatments with escitalopram, venlafaxine, or lithium in advance.

Further information of the patients at baseline: Mean age of 42 years (min: 19, max: 62), mean body-mass index (BMI) of 27, mean IDS-SR: 45 (min: 13, max: 65), mean IDS-C: 45 (min: 20, max: 67), mean HAM-D: 25 (min: 18, max: 39).

### Sample

The present example is based on a secondary analysis of 169 depressed patients who participated in the EMC trial (n = 889). Blood samples were available at baseline for 560 patients, from which the clearest responders (n = 86) and non-responders (n = 83) were selected. The selection was based on their response to antidepressant medication, measured by the HAM-D 17 after four weeks. In addition, depression severity was assessed at weekly intervals from baseline to week 8. The non-responders showed no improvement in depressive symptoms despite four weeks of antidepressant treatment; in the responders group, depressive symptoms decreased steadily during the course of treatment.

### Study procedures

At the screening visit, all participants underwent a detailed examination. Diagnoses were verified using the German version of the Mini International Neuropsychiatric Interview (M.I.N.I.) and the Structured Clinical Interview for DSM-IV Axis II Personality Disorders. Sociodemographic and clinical characteristics were assessed by self-report. Depression severity was monitored weekly from baseline to week 8 using the HAM-D 17 by trained and blinded raters. Morning blood samples were taken weekly in fasting patients before medication intake. If necessary, antidepressant premedication was washed out before the baseline visit. From baseline to day 14, antidepressant treatment for all patients was escitalopram (up to 20 mg per day). For the following six weeks, patients were treated according to the study protocol. No changes were made to concomitant medication for general medical conditions.

Spermidine and putrescine level determination in blood samples Plasma polyamines including spermidine and putrescine were quantified using liquid-chromatography coupled to mass spectrometry (LC-MS) according to Magnes et al., 2014, with modifications described in Costa-Machado et al., 2023. A modified carbonate buffer (1 M ammonium bicarbonate) that was adjusted to pH 10 using hydrochloric acid was used. Plasma was derived from whole blood by standard centrifugation procedures of anti-coagulated whole blood.

### Results

In the group of treatment responders, we observed an increase in spermidine levels, whereas a clear reduction in its levels was noted in the non-responder group over the course of the treatment (Fig. 1(b)). Putrescine, which is also a biogenic polyamine (and the metabolic precursor of spermidine), did not show a comparable progression (Fig. 1(c)).

### NON-PATENT REFERENCES

Costa-Machado, Luis Filipe, et al. "Peripheral modulation of antidepressant targets MAO-B and GABAAR by harmol induces mitohormesis and delays aging in preclinical models." Nature communications 14.1 (2023): 2779.
Fava, Maurizio. "Diagnosis and definition of treatment-resistant depression." Biological psychiatry 53.8 (2003): 649-659.
Fries, Gabriel R., et al. "Molecular pathways of major depressive disorder converge on the synapse." Molecular Psychiatry 28.1 (2023): 284-297.
Hamilton, Max. "A rating scale for depression." Journal of neurology, neurosurgery, and psychiatry 23.1 (1960): 56.
Magnes, Christoph, et al. "Polyamines in biological samples: Rapid and robust quantification by solid-phase extraction online-coupled to liquid chromatography-tandem mass spectrometry." Journal of chromatography A 1331 (2014): 44-51.
Qi, Guolian, et al. "Association between dietary spermidine intake and depressive symptoms among US adults: National Health and Nutrition Examination Survey (NHANES) 2005-2014." Journal of Affective Disorders 359 (2024): 125-132.
Rush, A. John, et al. "The inventory of depressive symptomatology (IDS): psychometric properties." Psychological medicine 26.3 (1996): 477-486.
Rush, A. John, Thomas Carmody, and Paul-Egbert Reimitz. "The Inventory of Depressive Symptomatology (IDS): clinician (IDS-C) and self-report (IDS-SR) ratings of depressive symptoms." International journal of methods in psychiatric research 9.2 (2000): 45-59.
Strawbridge, Rebecca, Allan H. Young, and Anthony J. Cleare. "Biomarkers for depression: recent insights, current challenges and future prospects." Neuropsychiatric disease and treatment (2017): 1245-1262.
Tadić, André, et al. "Rationale and design of the randomised clinical trial comparing early medication change (EMC) strategy with treatment as usual (TAU) in patients with Major Depressive Disorder-the EMC trial." Trials 11 (2010): 1-17.
Targum, Steven D., et al. "A novel peripheral biomarker for depression and antidepressant response." Molecular psychiatry 27.3 (2022): 1640-1646.
You, Zerui, et al. "The contribution of polyamine pathway to determinations of diagnosis for treatment-resistant depression: A metabolomic analysis." Progress in Neuro-Psychopharmacology and Biological Psychiatry 128 (2024): 110849.

## Claims

1. In-vitro use of spermidine, preferably the spermidine/putrescine ratio, as a biomarker for monitoring the treatment response in major depressive disorder (MDD); preferably wherein the biomarker is a urine biomarker, a saliva biomarker or a blood biomarker, especially a plasma biomarker.

2. A method for assessing a response to an MDD treatment in a patient having MDD, comprising the steps of
a) determining a first spermidine level in a first sample of the patient, wherein the first sample has been obtained from the patient prior to the MDD treatment, preferably wherein additionally a first putrescine level is determined in the first sample to obtain a first spermidine/putrescine ratio;
b) determining a second spermidine level in a second sample of the patient, wherein the second sample has been obtained while the patient is undergoing the MDD treatment or has undergone the MDD treatment, preferably wherein additionally a second putrescine level is determined in the second sample to obtain a second spermidine/putrescine ratio; and
c) correlating the first spermidine level with the second spermidine level, preferably by correlating the first spermidine/putrescine ratio with the second spermidine/putrescine ratio, to assess the response to the MDD treatment.

3. An antidepressant for use in treatment of MDD, wherein the spermidine level is used as a biomarker for treatment response, in particular according to the method of claim 2.

4. A method for treating MDD, comprising the steps of
a) obtaining a first sample from a patient having MDD,
b) determining a first spermidine level in the first sample, preferably wherein additionally a first putrescine level is determined in the first sample to obtain a first spermidine/putrescine ratio,
c) administering an MDD treatment to the patient,
d) obtaining a second sample from the patient,
e) determining a second spermidine level in the second sample, preferably wherein additionally a second putrescine level is determined in the second sample to obtain a second spermidine/putrescine ratio, and
f) correlating the first spermidine level with the second spermidine level, preferably by correlating the first spermidine/putrescine ratio with the second spermidine/putrescine ratio, to assess a response to the MDD treatment;
wherein step a) is performed before step c) and wherein step d) is performed after or concurrently with step c).

5. The method of claim 4, wherein, if said correlating indicates no clinical improvement of MDD, the patient is switched to another MDD treatment, and/or, if said correlating indicates clinical improvement of MDD, the MDD treatment is continued.

6. The use or method of any one of claims 1, 2, 4 and 5, wherein a spermidine level increase is indicative of the treatment response being a clinical improvement of the MDD, preferably wherein being a clinical improvement is defined as a decrease of the Hamilton Depression Rating Scale (HAMD)-17 score of at least 20%, preferably at least 30%, more preferably at least 40%, especially at least 50%.

7. The use or method of any one of claims 1, 2 and 4 to 6, wherein a spermidine level decrease or spermidine level being unchanged is indicative of the treatment response not being a clinical improvement of the MDD, preferably wherein not being a clinical improvement is defined as the HAMD-17 score being decreased less than 20%, the HAMD-17 score being unchanged or the HAMD-17 score being increased.

8. The use or method of any one of claims 1, 2 and 4 to 7, wherein the MDD treatment is selected from the group consisting of pharmacological therapy, neuromodulation therapies and psychotherapy.

9. The use or method of claim 8, wherein the MDD treatment is pharmacological therapy with an antidepressant.

10. The antidepressant for use according to claim 3 or the use or method of claim 9, wherein the antidepressant is selected from the group consisting of selective serotonin reuptake inhibitors (SSRIs), serotonin-norepinephrine reuptake inhibitors (SNRIs), serotonin modulators, monoamine oxidase inhibitors (MAOIs), tricyclic antidepressants, atypical antidepressants and N-methyl-D-aspartate (NMDA) receptor antagonists.

11. The antidepressant for use, or use or method according to claim 10, wherein the antidepressant is an SSRI, preferably selected from the group consisting of fluoxetine, sertraline, paroxetine, citalopram, escitalopram, and fluvoxamine, especially citalopram and escitalopram, or an SNRI, preferably selected from the group consisting of venlafaxine, desvenlafaxine, duloxetine, milnacipran, and levomilnacipran, especially venlafaxine and desvenlafaxine; optionally wherein the antidepressant is augmented by an antipsychotic, anticonvulsant or lithium, in particular lithium.

12. The method, use or antidepressant for use of any one of claims 1 to 11, wherein MDD is further defined by a HAMD-17 score of at least 18 and/or wherein MDD is further defined by a clinician-rated Inventory of Depressive Symptomatology (IDS-C) score of at least 20.

13. The method, use or antidepressant for use of any one of claims 1 to 12, wherein the time interval between the first sample and the second sample is at least one week, preferably at least two weeks, more preferably at least three weeks, especially at least four weeks.

14. The method, use or antidepressant for use of any one of claims 1 to 13, wherein the time interval between the initiation of MDD treatment and the second sample is at least one week, preferably at least two weeks, more preferably at least three weeks, especially at least four weeks.

15. The method, use or antidepressant for use of any one of claims 1 to 14, wherein the first sample and the second sample are blood samples, urine samples or saliva samples, preferably wherein the first sample and the second sample are plasma samples.
